# EUROPEAN PATENT APPLICATION

(11) **EP 4 755 442 A1**
(43) Date of publication of application: **10.06.2026**
(21) Application number: 24849428.8
(22) Date of filing: 17.07.2024
(51) Int. Cl.: A61N 1/32, A61N 1/04, A61N 1/06, A61N 1/36, A61N 1/40

(54) **SKIN CARE DEVICE USING ELECTRICAL STIMULATION**

(30) Priority: 28.07.2023 KR 20230098848; 28.07.2023 KR 20230098849
(71) Applicant: APR CO., LTD., Seoul 05551 (KR)
(72) Inventor: JI, Jong Chul, Seoul 05551 (KR); LEE, Gyoun Jung, Seoul 05551 (KR); PARK, Seung Woo, Seoul 05551 (KR)
(74) Representative: Herrmann, Daniel
(86) International application number: PCT/KR2024/010264
(87) International publication number: WO 2025/028859

(57) **Abstract**

The present invention relates to a portable skin care device using electrical stimulation that can easily contact body parts with relatively narrow and highly curved contact areas, such as the face, and can adjust the depth of stimulation. The present embodiment provides a skin care device using electrical stimulation for applying electrical stimulation to user skin, comprising: a head part contacting the user skin; and an electrode part consisting of a first electrode formed in a central region of a front surface of the head part and a second electrode formed along an edge region of the head part so as to surround the first electrode while being spaced apart from the first electrode by a predetermined interval, wherein the first electrode and the second electrode include one or more regions where a width of spaced interval varies.

## Description

### [Technical Field]

The present invention relates to a portable skin care device, and more particularly, to a portable skin care device using electrical stimulation that can easily contact body parts with relatively narrow and highly curved contact areas, such as the face, can adjust the depth of stimulation, and can improve cosmetic or massage effects.

### [Background Art]

As human skin ages, elasticity decreases, skin sags to form wrinkles, and prolonged exposure to ultraviolet rays causes the stratum corneum to thicken, making the skin less transparent, reducing the skin's blood circulation ability, and hindering the smooth supply of moisture and nutrients to the skin.

To solve these problems, skin beauty or massage methods are used, such as applying functional substances like nourishing creams to the skin or applying physical stimulation to the skin to facilitate blood circulation.

Recently, it has been known that high-frequency electrical stimulation to the skin promotes the absorption of creams applied to the skin and generates deep heat in the skin to promote blood circulation. Accordingly, various types of high-frequency electrical stimulators are being introduced.

Electrical stimulators using electrical energy are largely classified into two types according to the configuration of the electrodes. For example, the high-frequency stimulation method applied to electrical stimulators is classified into a monopolar method in which one electrode is contacted with the skin so that electrical energy flows back through the entire body together with a ground electrode, and a bipolar method in which two electrodes are contacted with the skin so that electrical energy applied to one electrode flows back to the other electrode arranged side by side.

The monopolar method is a method in which a single positive electrode in contact with the target area conducts electricity to another part of the body. It has the advantage of enabling deep penetration because the electric field between electrodes is formed widely, but it is not suitable for application to precise, local, or sensitive skin areas. On the other hand, in the bipolar method, since electrical energy does not flow back to areas outside the two electrodes, it does not affect tissues other than the treatment area, thereby preventing damage to unnecessary tissues, and can be efficiently applied to sensitive and thin skin.

It has been introduced through various papers that in bipolar electrical stimulators, the range in which the electric field is formed depends on the distance between both electrodes, and the depth to which electrical energy is delivered is different. In addition, electrical stimulators that control the penetration depth by adjusting the distance between invasive or non-invasive electrodes using this principle have been introduced through a number of patent documents.

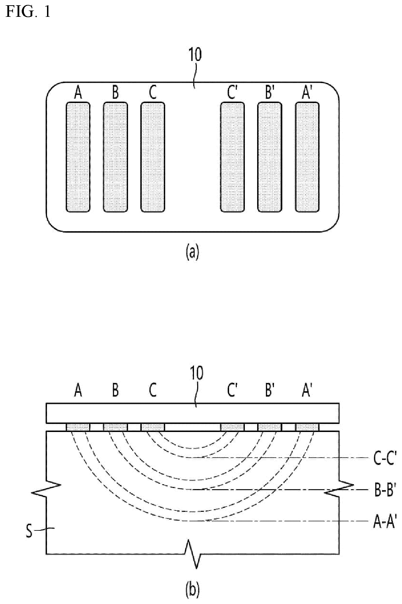

FIGS. 1 to 3 are diagrams showing various electrode structures of electrical stimulators according to the prior art and the flow of electrical energy accordingly.

In the electrical stimulator 10 of FIG. 1, three pairs of electrodes (A-A', B-B', C-C') are arranged side by side facing each other. In the electrode structure of FIG. 1, when three pairs of electrodes operate simultaneously, electrical energy penetrates over a three-stage range, having a wide application point. However, this may cause unnecessary or excessive stimulation when applied to facial areas where the skin (S) thickness is thin. In addition, in the electrode structure of FIG. 1, when one pair of electrodes among the three pairs is selectively activated, due to the nature of the sequential arrangement of the electrodes, it is impossible to penetrate electrical energy to a desired depth in a narrow area of skin, and there is a difficulty in contacting both electrodes completely to facial areas with many curves. This difficulty becomes more pronounced when the deepest penetration of electrical energy is required for a portion of the skin composed of a narrow area.

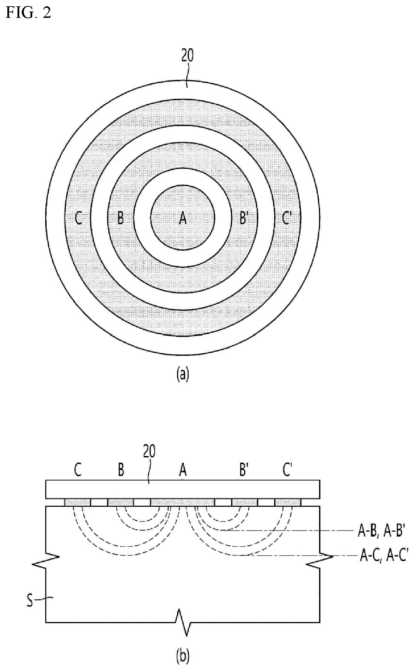

In the electrical stimulator 20 of FIG. 2, two ring-shaped electrodes (B, C) are radially arranged around a disc-shaped electrode (A). In the electrode structure of FIG. 2, in order to apply electrical stimulation to the skin, the 'A' electrode located in the center must be in contact with the skin. This electrode structure is suitable for use on skin with a relatively narrow area compared to the electrode structure of FIG. 1. However, the electrode structure of FIG. 2 has limitations in application to facial areas with many curves because sufficient contact area as large as the electrode head size must be secured to deliver stimulation to a deep penetration depth, and use is limited by the area of the electrode 'B-C' section even to stimulate a shallow penetration depth.

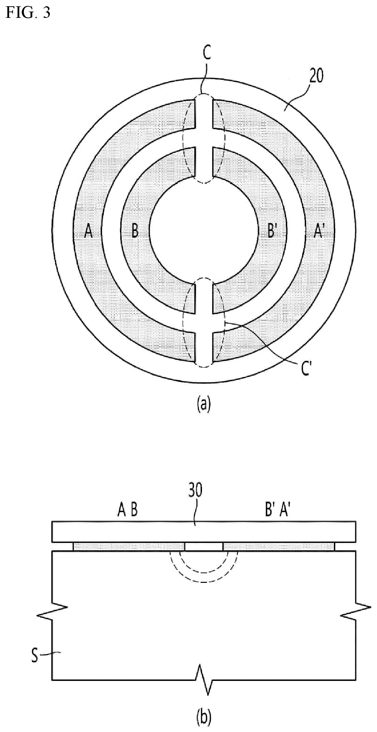

The electrical stimulator 30 of FIG. 3 is arranged in a structure in which two ring-shaped electrodes (A-A', B-B') face each other with the central portion separated. The electrode structure of FIG. 3 has a disadvantage in that an electric field is formed only in the regions (C, C') where each electrode faces each other, so that electrical stimulation is formed very shallowly, preventing efficient stimulation.

Generally, portable beauty devices or massage devices using high-frequency electrical stimulation used at home must be able to be used efficiently even on contact areas with many curves or extremely narrow areas such as the face, and must be able to adjust the intensity of stimulation to an appropriate level desired by the user for areas where stimulation is unnecessary.

However, conventional bipolar high-frequency electrical stimulators having the above electrode structures cannot be considered efficiently applied to a face having thin skin thickness and various curves within a narrow area, and the user cannot arbitrarily adjust the intensity of stimulation.

### [Prior Art Documents]

### [Patent Documents]

(Patent Document 1) KR 10-2023-0019432 A
(Patent Document 2) KR 10-2022-0155220 A
(Patent Document 3) KR 10-1503081 B1
(Patent Document 4) KR 10-2022-0119220 A
(Patent Document 5) US 8948838 B2
(Patent Document 6) JP 4102031 B2

### [Non-Patent Documents]

(Non-Patent Document 1) Elman M, Vider I, Harth Y, Gottfried V, Shemer A. Non-invasive therapy of wrinkles and lax skin using a novel multisource phase-controlled radio frequency system. J Cosmet Laser Ther. 2010 Apr;12(2):81-6. doi: 10.3109/14764171003706133. PMID: 20331345.
(Non-Patent Document 2) Royo de la Torre J, Moreno-Moraga J, Munoz E, Cornejo Navarro P. Multisource, Phase-controlled Radiofrequency for Treatment of Skin Laxity: Correlation Between Clinical and In-vivo Confocal Microscopy Results and Real-Time Thermal Changes. J Clin Aesthet Dermatol. 2011 Jan;4(1):28-35. PMID: 21278896; PMCID: PMC3030214.

### [Disclosure]

### [Technical Problem]

The present invention has been proposed to solve the above problems, and an object of the present invention is to provide a portable skin care device that can be efficiently used even on contact areas with many curves or extremely narrow areas, such as the face.

Another object of the present invention is to provide a portable skin care device that allows a user to adjust the intensity of stimulation to an appropriate level desired by the user for areas where stimulation is unnecessary.

### [Technical Solution]

An embodiment for solving the above problems provides a skin care device using electrical stimulation for applying electrical stimulation to user skin, comprising: a head part contacting the user skin; and an electrode part consisting of a first electrode formed in a central region of a front surface of the head part and a second electrode formed along an edge region of the head part so as to surround the first electrode while being spaced apart from the first electrode by a predetermined interval, wherein the first electrode and the second electrode include one or more regions where a width of the spaced interval varies.

Another embodiment for solving the above problems provides a skin care device using electrical stimulation for applying electrical stimulation to user skin, comprising: a head part contacting the user skin; and an electrode part consisting of a first electrode formed in a central region of a front surface of the head part and a second electrode formed along an edge region of the head part so as to surround the first electrode while being spaced apart from the first electrode by a predetermined interval, wherein the head part has the front surface contacting the skin in a circular shape but includes at least one corner part.

In addition, the second electrode may be formed on a front edge region and a side edge region of the head part.

### In addition, the corner part may be formed to have an internal angle of 150° or less.

In addition, the head part may have a polygonal structure on the front surface contacting the user skin.

### [Advantageous Effects]

According to the present invention configured as described above, since electrodes are also formed on the side surface of the head part and one or more corner parts are provided, it can be efficiently used even on areas with many curves or narrow contact areas, such as the face.

In addition, according to the present invention, electrical energy can penetrate to various depths depending on the position of the electrode, so the user can adjust the stimulation to be applied to an appropriate depth desired by the user while turning or tilting the electrical stimulator, thereby greatly improving user convenience.

### [Description of Drawings]

FIG. 1 is a diagram showing an example of an electrode structure and electrical energy flow of an electrical stimulator according to the prior art.
FIG. 2 is a diagram showing another example of an electrode structure and electrical energy flow of an electrical stimulator according to the prior art.
FIG. 3 is a diagram showing still another example of an electrode structure and electrical energy flow of an electrical stimulator according to the prior art.
FIG. 4 is a perspective view showing a skin care device according to an embodiment of the present invention.
FIG. 5 is a view showing the electrode structure of the head part of the skin care device of FIG. 4.
FIG. 6 is a view showing the flow of electrical energy of the skin care device of FIG. 4.
FIG. 7 is a view showing various examples of the head part of the skin care device according to an embodiment of the present invention.

### [Mode of Disclosure]

The technical problems achieved by the present invention and the practice of the present invention will become clear by the preferred embodiments described below. Hereinafter, preferred embodiments of the present invention will be described in detail with reference to the accompanying drawings.

The differences in the present embodiments described below should be understood as not being mutually exclusive. That is, specific shapes, structures, and characteristics described herein with respect to one embodiment may be implemented in other embodiments without departing from the technical spirit and scope of the present invention. The location or arrangement of individual components within each disclosed embodiment may be changed. In the drawings, similar reference numerals refer to the same or similar functions across various aspects, and length, area, thickness, etc., and their shapes may be exaggerated for convenience.

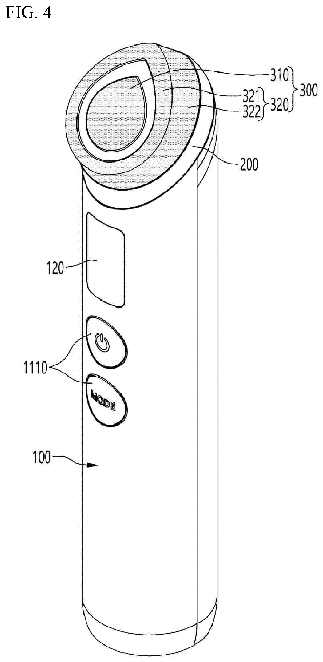

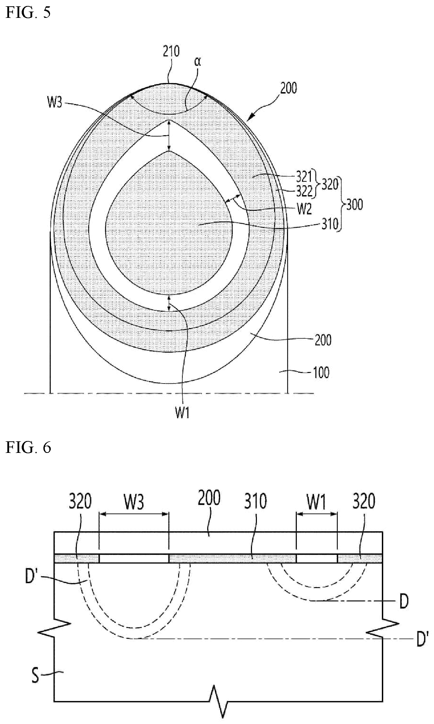

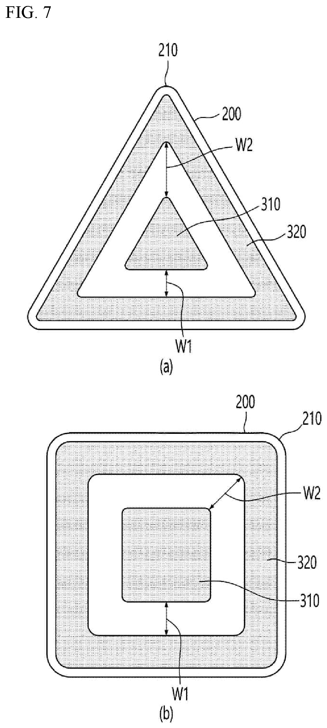

FIG. 4 is a perspective view showing a skin care device according to the present embodiment, FIG. 5 is a view showing the electrode structure of the head part of the skin care device of FIG. 4, FIG. 6 is a view showing the flow of electrical energy of the skin care device of FIG. 4, and FIG. 7 is a view showing various examples of the head part of the skin care device according to the present embodiment.

The skin care device according to the present embodiment is used for purposes such as beauty or massage by applying electrical stimulation to the epidermis or deep part of the skin. As shown in FIG. 4, it includes a handle part 100 provided for a user to hold the skin care device, a head part 200 provided at one end of the handle part 100, and an electrode part 300 provided on the front surface of the head part 200. Although not shown, various elements for driving the skin care device are installed in the internal spaces of the handle part 100 and the head part 200.

The handle part 100 is composed of a housing having a predetermined diameter and length so that a user can easily hold it with a hand, and provides a space in which driving elements are installed therein. In addition, an operation button 110 for user manipulation and a display panel 120 displaying driving information of the skin care device may be provided on one surface of the handle part 100.

The head part 200 is configured to contact the user's skin and is provided at one end of the handle part 100. The head part 200 may be composed of a housing integral with the handle part 100 or may be composed of a separate housing coupled to the handle part 100. The electrode part 300 formed on the surface of the head part 200 contacts the skin. The front surface of the head part 200 may form a circular shape, and the specific configuration of the head part 200 will be described later.

The electrode part 300 is formed on the surface of the head part 200 and generates deep heat inside the skin with high-frequency electrical signals transmitted from driving elements while in contact with the skin. The electrode part 300 may apply various types of electrical signals such as medium frequency, low frequency, or microcurrent as needed. The electrode part 300 includes a first electrode 310 and a second electrode 320.

The skin care device according to the present invention is a bipolar type device. When the first electrode 310 and the second electrode 320 contact the skin, electrical energy applied to any one of the first electrode 310 and the second electrode 320 flows back to the other electrode while applying electrical stimulation to the skin.

The first electrode 310 is formed in the central region of the front surface of the head part 200, and the second electrode 320 is formed along the periphery of the first electrode 310 while being spaced apart from the first electrode 310 by a predetermined interval. Accordingly, the first electrode 310 may have a circular shape or a ring shape, and the second electrode 320 has a ring shape.

In addition, the second electrode 320 is formed along the edge region of the head part 200, and is formed in an edge region including the front surface and the side surface of the head part 200. Specifically, the second electrode 320 is composed of a front region second electrode 321 formed on the front edge of the head part 200 and a side region second electrode 322 formed along the side edge. Therefore, since the skin care device can apply electrical stimulation even to skin contacting the side surface of the head part 200, it can apply electrical stimulation to curved parts or corner parts of the face as well.

The electrode part 300 generates deep heat inside the skin between the first electrode 310 and the second electrode 320. At this time, the depth at which deep heat is generated varies depending on the interval between the first electrode 310 and the second electrode 320. Considering this, the first electrode 310 and the second electrode 320 of the present embodiment include regions with varying intervals.

Specifically, as shown in FIG. 5, an interval (w1) between the first electrode 310 and the second electrode 320 at one side, an interval (w2) between the first electrode 310 and the second electrode 320 at another location, and an interval (w3) between the first electrode 310 and the second electrode 320 at yet another location may have different widths (w3 > w2 > w1). Accordingly, as shown in FIG. 6, in the region (w1) where the interval between electrodes is narrow, electrical energy penetrates into the skin (S) to a shallow depth (D), and in the relatively wide region (w3), electrical energy penetrates into the skin (S) to a deep depth (D').

With the electrode part 300 having such a configuration, the user can selectively apply deep and shallow stimulation by arbitrarily selecting the contact position of the head part 200, that is, the electrode part 300, by turning or tilting the stimulator while holding the skin care device. In addition, tooth pain (sensitive teeth sensation) sometimes occurs due to deep penetration of electrical stimulation during use of the skin care device. By selecting the electrode part 300 at a position where the interval between electrodes is narrow and contacting it to the skin, the user can prevent unnecessary stimulation such as tooth pain.

Referring again to FIGS. 4 and 5, the front part of the head part 200 forms a circular shape and includes at least one corner part 210. That is, the head part 200 may form a circular shape while forming a corner part 210 that is relatively pointed and has a predetermined internal angle on one side, thereby forming a teardrop shape as a whole. Generally, a circular head part is suitable for curved parts of the skin, and a triangular or square head part has the advantage of being able to efficiently apply electrical stimulation even to narrow areas such as corners or recessed parts of the skin. Therefore, the head part 200 having the corner part 210 as described above can be efficiently applied to a face having many curved parts, corners, or recessed parts, such as the face.

Specifically, the corner part 210 allows the electrode part 300 to easily contact the skin even in areas with many curves such as the face. On a human face, the area where the ala of the nose and the face surface connect is curved and connected at a predetermined angle, and the area around the nostrils or the eye area forms a relatively narrow region. The corner part 210 allows the electrode part 300 to be in close contact with the area where the ala of the nose and the face surface connect, around the nostrils, or the eye area without lifting from the face surface. That is, the user can efficiently apply electrical stimulation to these areas by contacting the electrode part 300 to curved parts or narrow parts of the face using the corner part 210 as the tip. At this time, the first electrode 310 and the second electrode 320 may be formed with the widest interval at the corner part 210.

The corner part 210 may have an internal angle (α) of a predetermined size, and it is preferable that the internal angle has a size of 150° or less. That is, the corner part 210 is formed so that the internal angle does not exceed 150°.

Meanwhile, when the head part 200 forms a circular shape, a separate corner part 210 may be shaped as shown in FIG. 5, but the head part 200 may form a polygonal shape such as a triangular shape or a square shape to form corner parts as shown in FIG. 7. In this case, three vertices or four vertices of the head part 200 form the corner parts 210, and the interval between the first electrode 310 and the second electrode 320 at the corner part 210 can be formed wider (w2 > w1).

In the detailed description of the present invention as described above, specific embodiments have been described. However, various modifications are possible without departing from the scope of the present invention. The technical spirit of the present invention should not be limited to the described embodiments of the present invention, but should be determined by the claims as well as equivalents to the claims.

### Description of Reference Numerals:

100: Handle part
200: Head part
210: Corner part
300: Electrode part
310: First electrode
320: Second electrode

## Claims

1. A skin care device using electrical stimulation for applying electrical stimulation to user skin, comprising:
a head part contacting the user skin; and
an electrode part consisting of a first electrode formed in a central region of a front surface of the head part, and a second electrode formed along an edge region of the head part so as to surround the first electrode while being spaced apart from the first electrode by a predetermined interval,
wherein the first electrode and the second electrode form one or more regions where a width of spaced interval varies.

2. The skin care device using electrical stimulation according to claim 1, wherein the second electrode is formed on a front edge region and a side edge region of the head part.

3. The skin care device using electrical stimulation according to claim 2, wherein the head part has a polygonal structure on the front surface contacting the user skin.

4. A skin care device using electrical stimulation for applying electrical stimulation to user skin, comprising:
a head part contacting the user skin; and
an electrode part consisting of a first electrode formed in a central region of a front surface of the head part, and a second electrode formed along an edge region of the head part so as to surround the first electrode while being spaced apart from the first electrode by a predetermined interval,
wherein the head part has the front surface contacting the skin in a circular shape but includes at least one corner part.

5. The skin care device using electrical stimulation according to claim 4, wherein the second electrode is formed on a front edge region and a side edge region of the head part.

6. The skin care device using electrical stimulation according to claim 4, wherein the corner part is formed to have an internal angle of 150° or less.

7. The skin care device using electrical stimulation according to claim 4, wherein the head part has a polygonal structure on the front surface contacting the user skin.
